Europäisches Patentamt

(19) European Patent Office

Office européen des brevets

(11) Publication number: **0 439 430 A2**

## (12) EUROPEAN PATENT APPLICATION

(21) Application number: 91810040.5

(51) Int. Cl.⁵: **A61L 15/44, A61K 9/70**

(22) Date of filing: 17.01.91

(30) Priority: 22.01.90 US 468388

(43) Date of publication of application:
31.07.91 Bulletin 91/31

(84) Designated Contracting States:
AT BE CH DE DK ES FR GB GR IT LI LU NL SE

(71) Applicant: CIBA-GEIGY AG
Klybeckstrasse 141
CH-4002 Basel (CH)

(72) Inventor: Mazzenga, Gerard Cesidio
11 Adam Place
New City, NY 10956 (US)
Inventor: Berner, Bret
16 Roxbury Road
Scarsdale, NY 10583 (US)

(54) Transdermal administration of zwitterionic drugs.

(57) Transdermal therapeutic systems containing zwitterionic drugs and methods of administering zwitterions transdermally are disclosed. The systems comprise a zwitterionic drug in a salt form and a solvent therefor.

EP 0 439 430 A2

## TRANSDERMAL ADMINISTRATION OF ZWITTERIONIC DRUGS

### Field of the Invention

The present invention relates to the area of transdermal administration of drug substances. It further relates to the area of zwitterionic drugs and methods of making such drugs transdermally administrable.

### Background of the Invention

Zwitterionic drugs have poor absorption through intact skin due to their rather large dipole moments and their resulting low lipid solubility. In general, salts of organic compounds have lower skin permeability than their corresponding free acids or bases. The unionized acids and bases can take advantage of lipophilic pathways through the skin that ionic species cannot. Typical non-zwitterionic acidic or basic substances can be placed in transdermal formulations at appropriate pH-values such that the active agent is substantially in the non-ionic form leading to enhanced absorption through human skin.

Zwitterionic drugs cannot be made non-ionic. At all pH-values, at least one ionic group is present. For example, at pH-values higher than the pKa of the acidic group(s) of a zwitterion, the acidic group is charged ; at pH-values lower than the pKa of the basic group(s) of a zwitterion, the basic group is charged. At pH-values close to the zwitterion pI, both groups are charged. Amino acid containing drugs, being the zwitterions of greatest interest, remain charged at all pH-values in the range of 2.0 to 11 only this pH-range is suitable for transdermal application. It would not be expected that one could achieve a suitable flux of a zwitterion through the skin to make transdermal administration thereof practical.

### Objects of the invention

One object of the present invention is to provide a transdermal composition having a zwitterionic active species which can be suitably administered transdermally.

Another object of the invention is to provide a method of administering zwitterionic drugs transdermally.

Still another object is to provide transdermal therapeutic systems (TTS) containing zwitterionic agents for absorption at efficacious doses.

### Summary of the invention

Surprisingly, these and other objects are achieved by the following invention which relates to a therapeutic system for the transdermal combined administration of a zwitterionic pharmaceutical active agent, consisting of :

(1) a closed outer layer which is impermeable to the constituents of the active ingredient formulation,

(2) a reservoir containing essential constituents of the active ingredient formulation, a vehicle and, optionally, a membrane,

(3) an adhesive layer and

(4) a peel-off protective layer on the adhesive layer, characterised in that the therapeutic system contains the zwitterionic pharmaceutical agent in salt form and optionally as vehicle an agent that enhances percentaneous absorption.

### Detailed description of the invention

The present invention resides in the fact that contrary to the low transdermal flux that would be expected from zwitterionic species regardless of pH, these drugs have much improved flux through skin when a salt form of the zwitterion is selected. The present invention teaches that salts of zwitterionic drugs uniquely have lower melting points than the parent zwitterion, higher solubility in essentially all solvents than the parent zwitterion, including solvents such as octanol (a model of skin lipids), and consequently higher transdermal fluxes (see Example 1, Table)

The zwitterionic materials within the scope of the invention include, without limitation : amino acids, peptides, polypeptides or proteins such as L-tyrosine, L-dopa, α-methyldopa, metirosine, levothyrosine, diiodotyrosine, liothyronin, melphalen, γ-aminobutyric acid, 4-amino-3-phenylbutyric acid, p-aminosalicyclic acid, baclofen, cefaclor, cefatrizine, cefroxadine, cefalexine, and especially 3-[(1-carboxy-3-phenylpropyl)amino]-2,3,4,5-tetrahydro-2-oxo-1H-benzazepine-1-acetic acid (benazeprilat) and 3-[(5-amino-1-carboxypen-

2

tyl)amino]-2,3,4,5-tetrahydro-2-oxo-1H-1-benzazepine-1-acetic acid (libenzapril) and enantiomers thereof.

The salt forms of the zwitterions that are suitable for use in the instant invention include the stoichiometric salt of the zwitterionic species with another entity having an acidic hydrogen and the resulting anion is not itself a zwitterion or an entity which is a suitable hydrogen acceptor group which is not a zwitterion. Alternatively, a cationic counterion may be selected. Particularly suitable are hydrohalide, especially hydrofluoride, hydrochloride, or hydrobromide ; lower alkyl sulfonate, such as methane sulfonate or ethane sulfonate ; dicarboxylate, such as the maleate ; and the alkali metal salts, such as the lithium or potassium salts. The hydrogen receptors most desirable for use in the invention include hydroxides, carbonates, and bicarbonates. Specifically useful anions include acetate, benzenesulfonate, benzoate, bicarbonate, bitartrate, bromide, camphorsulfonate, carbonate, chloride, citrate, dihydrochloride, EDT acetate, ethanedisulfonate, laurylsulfate, ethanesulfonate, fumarate, glucoheptonate, hexylresorcinate, hydrabamine, hydrobromide, hydrochloride, hydroxynaphthoate, iodide, hydroxyethanesulfonate, lactate, lactobionate, malate, maleate, mandelate, mesylate, methylbromide, methylnitrate, methylsulfate, mucate, napsylate, nitrate, pamoate, pantothenate, phosphate, diphosphate, polygalacturonate, salicylate, stearate, subacetate, succinate, sulfate, tannate, tartrate, and triethiodide. Specifically useful are cationic benzathine, chlorprocaine, choline, diethanolamine, ethylenediamine, methylglucamine, procaine, aluminium, calcium, lithium, magnesium, potassium, sodium, and zinc. Still other counteranions of use in the present invention include ; adipate, alginate, anhydromethylenecitrate, aspartate, bisulfate, butylbromide, camphorate, digluconate, dihydrobromide, disuccinate, glycerophosphate, hemisulfate, hydroiodide, methylenebissalicylate, naphthalenedisulfonate, oxalate, pectinate, persulfate, phenylethylbarbiturate, picrate, propionate, thiocyanate, tosylate, and undecanoate.

Within the above-mentioned species, for a given zwitterion, the greater the counterion's polarizability, the greater its charge distribution and the lesser its rigidity, the better the counterion is for the purposes of the present invention. It is assumed, that the salt formation in the manner described by the present invention results in a conversion of a large dipole moment in a free zwitterion into a much smaller one in the salt form allowing the molecule to take advantage of skin permeation pathways which would otherwise be foreclosed as a result of the increased solubility of the salt in both polar and nonpolar media.

The pH of the composition need only be maintained at a point which will maintain the integrity of the zwitterion/counter-ion salt association. Preferably, this pH is at least 1 pH unit away from the zwitterion's isoleectric point, more preferably at least 2 pH units from the zwitterion's isoelectric point. Advantageously, the pH range that is suitable for use in the invention is from about 2.0 to about 11.0, preferably about 3.0 to about 10, more preferably about 3.5 to about 9.0, still more preferably about 4.0 to about 8.5. Most preferably, the pH is maintained at or greater than the $pK_a$ of the zwitterion's basic group or at or below the $pK_a$ of the zwitterion's acidic group. More preferably, the pH is at least 0.5 pH units, more preferably at least 1.0 pH units, still more preferably at least 1.5 pH units and most preferably at least 2.0 pH units from the relevant $pK_a$.

The therapeutic system may be of any geometrical shape, e.g. by be oval, elliptical, circular, rectangular, optionally with rounded corners, oblong or rectangular with one or two rounded tabs. Other shapes are also possible.

The outer layer (1) consists of a material or of a combination of materials that must be impermeable to the constituents of the formulation contained in the reservoir (2). It serves as a protecting and supporting layer. To produce the outer layer, it is possible to use high or low pressure polymers such as polyethylene, polypropylene, polyvinyl chloride, polyethylene terephthalate or also cellulose acetate or vinyl acetate/vinyl chloride copolymers and combinations, especially composite foils thereof. An impermeable, flexible outer layer that conforms to the shape of the part of the body to which the plaster is applied is preferred.

-The reservoir (2) is situated between the outer layer (1) and the adhesive layer (3) and contains essential constituents of the active ingredient formulation, e.g. the active zwitterionic ingredients in salt form optionally together with vehicle and/or the penetration enhancer. In addition, the reservoir may contain polymeric materials for the formation of a porous or permeable membrane.

The reservoir (2) may also contain other optional ingredients which will further enhance the delivery of the desired species. Such optional items include, without limitation, additional flux enhancers, preservatives, stabilizers, buffers, thickeners, carriers and fillers, which are compatible with the requisite pH range and selection of counterion outlined by the present invention.

A vehicle for the active species may also usually be present ; however, if the active agent or its salt is a liquid, then a solvent is not essential. In some instances, a vehicle may also act as a flux enhancer. Typical vehicles for use in the instant invention include, without limitation ; water ; $C_1$-$C_{10}$alkanols such as ethanol, propanol, isopropanol, propylene glycol, glycerol, and other hydrogen bonding solvents, such as DMSO, dimethylformamide, ethyl acetate, fatty acid esters, pyrrolidones such as 2-pyrrolidone, N-(2-hydroxyethyl)pyrrolidone, and N-methylpyrrolidone, polyalkanols, 1-n-dodecylazacycloheptan-2-one or eucalyptol (1,8-cineol). When vehicles are present, they are generally mixtures of water with at least one non-water solvent mentioned above.

These mixtures may be from 3 % to 97 %, preferably 20 % to 80 %, more preferably 20 % to 60 % non-aqueous solvent component.

The reservoir (2) contains the salt form of the zwitterionic agent, which may be formed in situ, in an amount which would deliver a therapeutically effective amount thereof over a period of time. Such amounts can be readily determined from the length of time the zwitterion is to be administered, the flux thereof through the skin and transdermal layers which separate the drug depot from the skin surface, the size of the contact area of the transdermal with the patient, etc, all of which would be readily within the abilities of one of ordinary skill in the art.

The active ingredient formulation can be prepared according to standard formulating techniques available to those skilled in the relevant art. The formulation can be placed into the transdermal device by the same techniques used for loading any other transdermal system with drug formulation.

Suitable transdermal therapeutic systems which may be applied are for example those disclosed in US patent specifications 3 598 122, 3 598 123, 3 797 494 and 4 064 084, preferably the systems disclosed in DE-A 26 04 718 and in US patent specifications 4 031 984 and 4 262 003, or described by H. Asche in Schweiz. Rundschau Med. (Praxis) 74, No. 11, 257-260 (1985), e.g. matrix or monolith systems or membrane-controlled systems. In this connection it must be emphasised that such application is not limited to the transdermal therapeutic systems disclosed and described in the aforementioned publications.

The reservoir (2) may contain a microporous membrane, the pores of which are filled with the above vehicle and which controls the rate at which the drug is released to the skin. The flux of drug through the semipermeable layer and the contact surface area of the membrane must be chosen such that the drug is released to the skin from the reservoir layer at substantially constant rate in the range from c. 0.3 to 5 $\mu$g/h. The semipermeable membrane is made from polymeric materials through which the drug can diffuse. Polymers suitable for making such membranes are described in the publications previously referred to, e.g. polypropylene, polyacrylates, polyvinyl chloride, polyester, siliconated polyester laminates, cellulose acetate, cellulose nitrate, polyacrylonitrate, copolymers of ethylene with other monomers, e.g. vinyl acetate, or organopolysiloxane rubber.

The adhesives that can be used in dermatology are suitable for the adhesive layer (3). Suitable adhesives are, for example, adhesive formulations of acrylic acid resins or methacrylic acid resins, e.g. polymers of acrylic acid or methacrylic acid esterified by alcohols such as n-butanol, n-pentanol, isopentanol, 2-methylbutanol, 1-methylbutanol, 1-methylpentanol, 2-methylpentanol or 3-methylpentanol, 2-ethylbutanol, isooctanol, n-decanol or n-dodecanol, or copolymers of these acrylic acid or methacrylic acid esters with monomers containing ethylene groups, such as acrylic acid itself, methacrylic acid, acrylamide, methacrylamide, N-alkoxymethacrylamide, N-alkoxymethylmethacrylamide, N-tert.-butylamide, itaconic acid, vinyl acetate, N-branched alkylmaleic acid amide in which the branched alkyl group has from 10 to 24 carbon atoms, glycol diacrylates or mixtures thereof, natural or synthetic rubber such as styrenebutadiene, butyl ether, neoprene, polyisobutylene, polybutadiene and polyisoprene, polyvinyl acetate, urea formaldehyde resins, resorcinol formaldehyde resins, cellulose derivatives such as ethylcellulose, methylcellulose, nitrocellulose, cellulose acetate butyrate and carboxymethylcellulose, and also natural gums such as guar, acacia, pectin, starch, dextrin, albumin, gelatin, casein etc.. Thickeners and stabilisers may also be added to the adhesives mentioned.

The protective layer (4) is removed before application. It consists of materials that are impermeable to the constituents of the reservoir layer (2). It is possible to use the same materials as those used for producing the outer layer (1), and also metal foils, for example thin aluminium foils. Organic polymers are rendered capable of being peeled off the adhesive layer (3) by suitable surface treatment, for example silicone treatment.

Having fully described the instant invention, the following non-limiting examples are presented to more clearly set it forth.

Example 1 : The solubilities of free zwitterion and enhanced solubilities of various salts thereof are reported in various degrees in the following solvents : water, ethanol, octanol, chloroform and human stratum corneum. Permeation rates are also shown for these zwitterionic compounds and enhanced fluxes of the salts thereof through materials including polyurethane. While such materials are impractical for transdermal devices for free zwitterions, these materials are practical for the transdermal delivery of salts of zwitterions. The solubility in water is given as a model or polar solvents, while the octanol and chloroform are given as models for skin lipids. The critical factor for inclusion of a salt within the invention is that relative to its non salt zwitterion parent compound, the salt form of the invention has a greater solubility in both polar and lipid skin models.

EP 0 439 430 A2

| Zwitterionic compounds and corresponding salts | mp/dec C | Solubility in water at 25 deg. C. (μg/ml) | R.E.* | Solubility in ethanol at 25 deg. C. (μg/ml) | R.E.* | Solubility in octanol at 25 deg. C. (μg/ml) | R.E.* | Solubility in chloroform at 25 deg. C. (μg/ml) | R.E.* |
|---|---|---|---|---|---|---|---|---|---|
| Phenylalanine (Phe) | 285 | 25321.8 | 1.00 | 234 | 1.00 | 12.3 | 1.00 | 0.21 | 1.00 |
| Phe. hydrofluoride | 254 | 24837.4 | 0.98 | 411.66 | 1.76 | 52.24 | 4.25 | 0.29 | 1.38 |
| Phe. hydrochloride | 240 | 129008.6 | 5.09 | 24053.91 | 102.79 | 2601.4 | 211.50 | 1.61 | 7.67 |
| Phe. hydrobromide | 230 | 95690.8 | 3.78 | 107145.9 | 457.89 | 40231.88 | 3270.88 | 58.26 | 277.43 |
| | | | | | | | | | |
| Baclofen (Bac) | 206 | 6828.7 | 1.00 | 1144.1 | 1.00 | 22.2 | 1.00 | 0.22 | 1.00 |
| Bac. methane sulfonate | 137 | 8826.9 | 1.29 | 170840 | 149.32 | | | 0.087 | 0.40 |
| Bac. ethane sulfonate | 140 | 38889.8 | 5.70 | 237181 | 207.31 | | | 11.17 | 50.77 |
| Bac. propane sulfonate | 166 | 24425.7 | 3.58 | 107317 | 93.80 | | | 8.74 | 39.73 |
| Bac. pentane sulfonate | 144 | | | 61650.3 | 53.88 | 576.4 | 25.96 | 37.4 | 170.00 |
| Bac. lithium salt | 214 | 21719.1 | 3.18 | 57837.3 | 50.55 | | | 6.93 | 31.50 |
| Bac. sodium salt | 252 | 21379.7 | 3.13 | 529056 | 462.42 | | | 3.47 | 15.77 |
| Bac. potassium salt | 181 | | | | | | | | |
| Bac. hydrochloride | 195 | 244622.5 | 35.82 | 37498.9 | 32.78 | 324.3 | 14.61 | 5.34 | 24.27 |
| Bac. hydrobromide | 174 | 351946.8 | 51.54 | 129569.7 | 113.25 | 477.05 | 21.49 | 13.7 | 62.27 |
| | | | | | | | | | |
| Libenzapril | 218 | 491500 | 1 | 190.7 | 1 | 23.36 | 1 | | |
| Libenzapril monomaleate | 144 | 623900 | 1.26 | 248.82 | 1.30 | 13.96 | 0.5976 | | |
| Libenzapril dihydrochloride | 201 | 1127800 | 2.29 | 1890.75 | 9.91 | 87.8 | 3.759 | | |
| Libenzapril dihydrobromide | 183 | 1423000 | 2.9 | 45026.9 | 235.90 | 506.7 | 21.69 | | |
| | | | | | | | | | |
| Benazeprilat | 264 | | | 120.89 | 1.00 | | | | |
| Benazeprilat dilithium salt | 172 | | | 27975.61 | 231.41 | | | | |
| Benazeprilat dipotassium salt | d<161 | | | 34193.51 | 282.85 | | | | |

*Relative enhancement
Average of values measured in triplicate

| Zwitterionic compounds and corresponding salts | Solubility in poly-urethane ($\mu$g/ml) x10^-3 | R.E.* | Solubility in 35 % ethyl vinyl acetate ($\mu$g/ml) x10^-3 | R.E.* | Permeation rate through poly-urethane ($\mu$g/cm$^2$/hr) | R.E.* | Solubility in human stratum corneum ($\mu$g/ml) | R.E.* | Permeation rate through human epidermis ($\mu$g/cm$^2$/hr) | R.E.* |
|---|---|---|---|---|---|---|---|---|---|---|
| Phenylalanine (Phe) |  |  |  |  | 1.92 | 1.00 |  |  | 1.02 | 1.00 |
| Phe. hydrofluoride |  |  |  |  | 4.68 | 2.44 |  |  | 2.14 | 2.10 |
| Phe. hydrochloride |  |  |  |  | 335.9 | 174.95 |  |  | 64.37 | 63.11 |
| Phe. hydrobromide |  |  |  |  | 1516.23 | 789.70 |  |  | 77.53 | 76.01 |
|  |  |  |  |  |  |  |  |  |  |  |
| Baclofen (Bac) | 1.95 | 1.00 |  |  | 5.32 | 1.00 | 3.11 | 1.00 | 0.118 | 1.00 |
| Bac. methane sulfonate | 10.96 | 5.62 |  |  | 287.9 | 54.12 |  |  |  |  |
| Bac. ethane sulfonate | 21.90 | 11.23 |  |  | 211.9 | 39.83 |  |  |  |  |
| Bac. propane sulfonate | 11.60 | 5.95 |  |  | 233.9 | 43.97 |  |  |  |  |
| Bac. pentane sulfonate |  |  |  |  | 220.2 | 41.30 |  |  |  |  |
| Bac. lithium salt | 28.90 | 14.82 |  |  | 128.3 | 24.12 |  |  |  |  |
| Bac. sodium salt | 12.00 | 6.15 |  |  | 605.3 | 113.78 |  |  |  |  |
| Bac. potassium salt | 42.40 | 21.74 |  |  | 587.8 | 110.49 |  |  |  |  |
| Bac. hydrochloride | 178 | 91.28 |  |  | 273.7 | 51.45 | 214.69 | 69.03 |  |  |
| Bac. hydrobromide | 640 | 328.2 |  |  | 738.9 | 138.89 | 292.2 | 93.95 | 25.49 | 216.02 |
|  |  |  |  |  |  |  |  |  |  |  |
| Libenzapril | 0.0039 | 1 | 0.0048 | 1 | 0.538 | 1.00 |  |  |  |  |
| Libenzapril monomaleate | 0.0105 | 2.69 | 0.0156 | 3.25 | 3.47 | 6.45 |  |  |  |  |
| Libenzapril dihydrochloride | 0.0084 | 2.15 | 0.042 | 8.75 | 13.34 | 24.80 |  |  |  |  |
| Libenzapril dihydrobromide | 0.29 | 74.35 | 0.336 | 70 | 65.31 | 121.39 |  |  |  |  |
|  |  |  |  |  |  |  |  |  |  |  |
| Benazeprilat |  |  |  |  | 0.311 | 1.00 |  |  | 0.1409 | 1.00 |
| Benazeprilat dilithium salt |  |  |  |  | 47.76 | 153.57 |  |  | 8.95 | 63.52 |
| Benazeprilat dipotassium salt |  |  |  |  | 75.01 | 241.19 |  |  | 20.56 | 145.92 |

*Relative enhancement
Average of values measured in triplicate

EP 0 439 430 A2

As is readily apparent, the salt form of the zwitterionic substance is generally more soluble than the free zwitterion in the solvent with the one exception of baclofen methane sulfonate in chloroform. Furthermore, the salt forms all have greater permeation rates through polyurethane than free zwitterions. Finally, of the tests conducted, the zwitterionic salts have significantly greater fluxes through stratum corneum and epidermis than their free zwitterion counterparts.

Example 2 : A release liner of polyester coated on one side with silicone is laid down with the silicone face up. Over the periphery of the system a 1 mil layer of Kraton™ (styrene/polyethylene/butylene block copolymer) medical adhesive is placed on the release liner. A 2 mil microporous polyethylene membrane is placed over the adhesive layer. A composition of 40 % v/v ethanol/water saturated with benazeprilat (170 mg/ml) and thickened with 1 % Klucel® (hydroxypropyl cellulose) is dropped onto the microporous membrane in an amount to insure a total fill volume of 600 mg/10 cm² of surface area through which the drug will migrate to the patient. Finally a polyester-EVA colaminate is prepared and laid down on the thickened composition with the EVA side facing the drug composition. The entire unit is heat sealed around the perimeter to produce the finished transdermal product.

Example 3 : A release liner of polyester coated on one side with silicone is laid down with the silicone face up. Over the periphery of the system a 1 mil layer of Kraton™ medical adhesive is placed on the release liner. A 2 mil microporous polyethylene membrane is placed over the adhesive layer in one region and a 2 mil low density polyethylene (non-microporous) membrane is placed over the adhesive in a second region. A composition of 40 % v/v ethanol/water saturated with baclofen (400 mg/ml) and thickened with 1 % Klucel is dropped onto the microporous membrane in an amount to insure a total fill volume of 600 mg/10 cm² of surface area through which the drug will migrate to the patient.

Simultaneously a composition of nicotine (60 mg in 32 mg of sodium hydroxide in 0.35 mg water with 6 mg of Carbopol 934 P [polyacrylic acid]) is dropped onto the non-microporous membrane. Finally, a polyester-EVA colaminate is prepared and laid down on the drug compositions with the EVA side facing the drug compositions. The entire unit is heat sealed around the perimeter and between the two drug formulations to produce the finished transdermal product.

## Claims

1. A therapeutic system for the transdermal combined administration of a zwitterionic pharmaceutical active agent, consisting of :
   (1) a closed outer layer which is impermeable to the constituents of the active ingredient formulation,
   (2) a reservoir containing essential constituents of the active ingredient formulation, a vehicle and, optionally a microporous membrane,
   (3) an adhesive layer and
   (4) a peel-off protective layer on the adhesive layer, characterised in that the therapeutic system contains the zwitterionic pharmaceutical agent in salt form and optionally as vehicle an agent that enhances percutaneous absorption.

2. The system according to claim 1 wherein the zwitterionic active agent is selected from the group consisting of amino acids, peptides, polypeptides and proteins and ACE inhibitors.

3. The system according to claim 1 wherein the salt form of said zwitterionic active agent is selected from the group consisting of hydrohalide, lower alkyl sulfonate, lower alkyl dicarboxylate, and alkali metal salt.

4. The system according to claim 1 wherein the vehicle is selected from the group consisting of water, $C_1$-$C_{10}$alkanols, and other hydrogen bonding solvents.

5. The system according to claim 1 wherein the zwitterionic active agent is selected from benazeprilat.

6. The system according to claim 1 wherein the zwitterionic active agent is selected from libenzapril.

7. A method of preparing a therapeutic system according to claim 1, characterised in that the constituents of the therapeutic system are applied in succession to the peel-off protective layer (4) and are optionally welded to one another.

## Claims for the following Contracting States : GR and ES

1. A process for the preparation of a therapeutic system for the transdermal combined administration of a zwitterionic pharmaceutical active agent, consisting of :

   (1) a closed outer layer which is impermeable to the constituents of the active ingredient formulation,
   (2) a reservoir containing essential constituents of the active ingredient formulation, a vehicle and, optionally a microporous membrane,
   (3) an adhesive layer and
   (4) a peel-off protective layer on the adhesive layer, characterised in that the therapeutic system contains the zwitterionic pharmaceutical agent in salt form and optionally as vehicle an agent that enhances percutaneous absorption, characterised in that the constituents of the therapeutic system are applied in succession to the pee-off protective layer (4) and are optionally wlded to one another.

2. A process for the preparation of the system according to claim 1 wherein the zwitterionic active agent is selected from the group consisting of amino acids, peptides, polypeptides and proteins and ACE inhibitors.

3. A process for the preparation of the system according to claim 1 wherein the salt form of said zwitterionic active agent is selected from the group consisting of hydrohalide, lower alkyl sulfonate, lower alkyl dicarboxylate, and alkali metal salt.

4. A process for the preparation of the system according to claim 1 wherein the vehicle is selected from the group consisting of water, $C_1$-$C_{10}$alkanols, and other hydrogen bonding solvents.

5. A process for the preparation of the system according to claim 1 wherein the zwitterionic active agent is selected from benazeprilat.

6. A process for the prepraration of the system according to claim 1 wherein the zwitterionic active agent is selected from libenzapril.